(19) Europäisches Patentamt / European Patent Office / Office européen des brevets

(11) EP 2 773 429 B1

(12) **EUROPÄISCHE PATENTSCHRIFT**

(45) Veröffentlichungstag und Bekanntmachung des Hinweises auf die Patenterteilung:
**04.01.2017 Patentblatt 2017/01**

(21) Anmeldenummer: **12778073.2**

(22) Anmeldetag: **09.10.2012**

(51) Int Cl.:
***A61Q 5/10*** *(2006.01)* ***A61Q 5/08*** *(2006.01)*
***A61K 8/81*** *(2006.01)*

(86) Internationale Anmeldenummer:
**PCT/EP2012/069979**

(87) Internationale Veröffentlichungsnummer:
**WO 2013/064341 (10.05.2013 Gazette 2013/19)**

(54) **FARBINTENSIVIERUNG DURCH POLYQUATERNIUM**

COLOR INTENSIFICATION BY POLYQUATERNIUM

INTENSIFICATION DE LA COULEUR AU MOYEN D'UN POLYQUATERNIUM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2011 DE 102011085754**

(43) Veröffentlichungstag der Anmeldung:
**10.09.2014 Patentblatt 2014/37**

(73) Patentinhaber: **Henkel AG & Co. KGaA**
**40589 Düsseldorf (DE)**

(72) Erfinder:
- **SCHETTIGER, Norbert**
  **40723 Hilden (DE)**
- **JANSSEN, Frank**
  **41470 Neuss (DE)**

(56) Entgegenhaltungen:
**WO-A1-2005/087191 WO-A2-2010/133573**

- **DATABASE GNPD [Online] MINTEL; Februar 2009 (2009-02), "Conditioner", XP002699621, Database accession no. 1048206**
- **Anonymous: "Polyquaternium-91", Personal Care Products Council , XP002699622, Gefunden im Internet: URL:http://gov.personalcarecouncil.org/jsp /gov/IngredientDetail.jsp?monoid=23746 [gefunden am 2013-06-26]**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 773 429 B1

## Beschreibung

Die vorliegende Erfindung betrifft Mittel zum Färben und/oder Aufhellen von keratinischen Fasern, insbesondere menschlichen Haaren, welche spezielle kationische Acrylsäureester-Copolymere enthalten. Weiterhin betrifft die Erfindung die Verwendung dieser Mittel zur Verbesserung des Farbaufzugs bei der Färbung von keratinischen Fasern, deren Verwendung zur Verbesserung der Waschstabilität von gefärbten keratinischen Fasern sowie ein entsprechendes Verfahren. Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Neben den Blondiermitteln, die eine oxidative Aufhellung der Haare durch Abbau der natürlichen Haarfarbstoffe bewirken, sind im Bereich der Haarfärbung im wesentlichen drei Typen von Haarfärbemitteln von Bedeutung: Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Bei, insbesondere mehrfacher, Anwen-dung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muß. Bei Personen mit ursprünglich mittel-blondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Nach dem Färbeprozeß ist das Haar über einen langen Zeitraum den verschiedensten Umweltbelastungen ausgesetzt. Diese reichen von der täglichen Bewitterung der Haare, beispielsweise durch Sonnenstrahlen und Haarwäschen, über mechanische, durch das Frisieren hervorgerufene Beanspruchungen bis zu chemischen Einflüssen, sofern der Konsument die Haare einem nachfolgenden Haarfärbe- oder Formungsprozess unterwirft. Die Umwelteinflüsse haben nicht nur auf die Haarstruktur selbst, sondern auch auf die nach dem Färbeprozess im Haar befindlichen Farbstoffe große Auswirkungen. Die Farbstoffe können durch die Belichtung ausbleichen, oder durch Schweiß oder Shampoonieren aus dem Haar heraus gewaschen werden. Diese Eigenschaften werden als die Echtheitseigenschaften der Farbstoffe bezeichnet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen. Sowohl das Auftreten von Farbverschiebungen als auch insbesondere das durch Auswaschen bedingte Verblassen der Färbungen ist vom Verbraucher unerwünscht.

Es gibt verschiedene Möglichkeiten, den durch wiederholtes Waschen bedingten Intensitätsverlust von Haarfärbungen zu reduzieren. Durch Anstreben eines von Anfang an möglichst intensiven Färbeergebnisses lässt sich der vom Verbraucher wahrgenommene Intensitätsabfall hinauszögern. Eine zweite Möglichkeit ist die Verbesserung des Farberhalts durch den Einsatz von Wirkstoffen, welche den Auswaschvorgang der Farbstoffe inhibieren bzw. reduzieren. Von besonderem Vorteil ist das Auffinden eines Wirkstoffes bzw. einer Wirkstoffkombination, welche sowohl das Farbaufzugsvermögen der Farbstoffe während des Färbeprozesses verbessert als auch deren späteres Auswaschen beim nachfolgenden Shampoonieren minimiert.

Die Aufgabe der vorliegenden Erfindung ist daher die Bereitstellung von Mitteln zum Färben und/oder Aufhellen von keratinischen Fasern, welche den Farbaufzug während der Färbung verbessern und die Penetration von Farbstoffen bzw. Farbstoffvorprodukten in die keratinischen Fasern verstärken. Weiterhin soll ein Auswaschen der Farbstoffe im Zuge wiederholter Shampoonierungen verhindert oder minimiert werden. Die erfindungsgemäßen Mittel sollen Farbintensität und Waschechtheit verbessern, ohne hierbei die Nachteile zu besitzen, die den aus dem Stand der Technik

bereits bekannten Färbemitteln zu eigen sind.

Darüber hinaus sollen mit diesen Mitteln auch Färbungen erzielt werden, die im Hinblick auf ihre weiteren Echtheitseigenschaften, wie beispielsweise ihre Licht-, Reib-, Schweiß- und Kaltwellechtheit, ein vorteilhaftes anwendungstechnisches Profil aufweisen. Schließlich ist besonders wünschenswert, Färbemittel mit gutem Egalisiervermögen bereitzustellen.

Im Kosmetikmarkt kommt der Optimierung des Farberhalts eine große Bedeutung zu, verschiedene Farbschutz-Shampoos oder Conditioner exisiteren bereits im Markt. Aus dem Stand der Technik sind diverse Substanzen bekannt, die zum Farbschutz von bereits coloriertem Haar eingesetzt werden. Insbesondere Polymere finden mit dieser Zielsetzung Anwendung.

"Silicones Used in Permanent and Semi-Permanent Hair Dyes to Reduce the Fading and Color Change Process of Dyed Hair Occuring by Wash-Out or UV Radiation", J. Cosmetic Sci., (2004) 55 (Supplement), S. 123-131, beschreibt den Einsatz von Silikonen zur Verbesserung des Farbrückhaltes. US 7 066 966 und US 7 147 672 offenbaren eine oxidative Färbezusammensetzung mit guten Waschechtheiten enthaltend ein kationisches Polyvinyllactam. US 2011/0219552 A1 offenbart eine Methode zum Schutz von gefärbtem Haar gegenüber dem Auswaschen durch Anwendung von hydrophobierten kationischen Polymeren. Schließlich betrifft die US 2011/0044924 Mittel und Verfahren zur Erhöhung der Farbintensität und zum Farbschutz von gefärbten Haaren, welche quartäre Ammoniumsalze enthalten.

Der Einsatz dieser aus dem Stand der Technik bekannten Polymere ist jedoch oftmals auch mit Nachteilen verbunden. In überraschender Weise hat sich nun gezeigt, dass Haarbehandlungsmittel enthaltend spezielle kationische Acrylsäureester-Copolymere die oben beschriebene Aufgabenstellung erfüllen, ohne mit den aus dem Stand der Technik bekannten Nachteilen behaftet zu sein. Ein erster Gegenstand der vorliegenden Erfindung sind daher Mittel zum Färben und/oder Aufhellen von keratinischen Fasern, insbesondere menschlichen Haaren, enthaltend in einem kosmetischen Träger

(a) mindestens ein kationisches Acrylsäureester-Copolymer enthaltend mindestens eine Struktureinheit der allgemeinen Formel (I), mindestens eine Struktureinheit der allgemeinen Formel (II) und mindestens eine Struktureinheit der allgemeinen Formel (III),

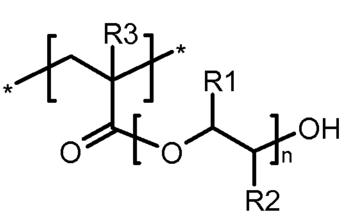

(I)

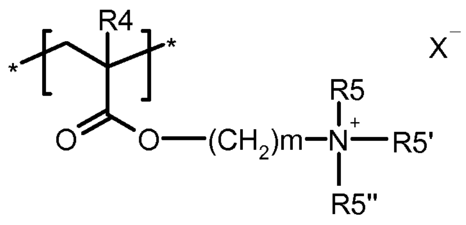

(II)

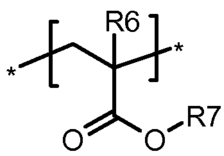

(III)

worin

| | |
|---|---|
| R1, R2, R3, R4, R6 | unabhängig voneinander für ein Wasserstoffatom oder eine $C_1$-$C_6$-Alkylgruppe stehen, |
| R5, R5', R5" | unabhängig voneinander für eine $C_1$-$C_{20}$-Alkylgruppe, eine $C_2$-$C_6$-Alkenylgruppe oder eine $C_2$-$C_6$-Hydroxyalkylgruppe stehen, |
| R7 | für eine $C_1$-$C_6$-Alkylgruppe, eine $C_2$-$C_6$-Hydroxyalkylgruppe oder eine $C_2$-$C_6$-Polyhydroxyalkylgruppe steht, |
| n | für eine ganze Zahl von 1 bis 50 000 steht, |
| m | für eine ganze Zahl von 2 bis 6 steht, |
| $X^-$ | für ein physiologisch verträgliches Anion steht, und |

(b) mindestens eine farbverändernde Verbindung.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Als ersten wesentlichen Inhaltsstoff enthalten die erfindungsgemäßen Mittel mindestens ein kationisches Acrylsäureester-Copolymer, enthaltend mindestens eine Struktureinheit der allgemeinen Formel (I), mindestens eine Struktureinheit der allgemeinen Formel (II) und mindestens eine Struktureinheit der allgemeinen Formel (III).

Im Folgenden werden Beispiele für die in den Formeln (I), (II) und (III) genannten Substituenten R1, R2, R3, R4, R5, R5', R5", R6 und R7 genannt:

Beispiele für $C_1$-$C_6$-Alkylgruppen sind $-CH_3$, $-CH_2CH_3$, $-CH_2CH_2CH_3$, $-CH(CH_3)_2$, $-CH_2CH_2CH_2CH_3$,$-CH_2CH(CH_3)_2$, $-CH(CH_3)CH_2CH_3$, $-C(CH_3)_3$, $-(CH_2)_4CH_3$, $-(CH_2)_5CH_3$. Besonders bevorzugte Alkylreste sind Methyl und Ethyl. Beispiele für $C_6$-$C_{20}$ Alkylgruppen sind Hexyl, Heptyl, Octyl, Nonyl, Decyl, Dodecyl, Tetradeyl, Pentadecyl, Hexadecyl, Heptadecyl, Octadecyl, Nonadecyl und Eicosyl. Beispiele für $C_2$-$C_6$-Alkenylgruppen sind Vinyl, Prop-2-enyl (Allyl), 2-Methyl-prop-2-enyl, But-3-enyl, But-2-enyl, Pent-4-enyl oder Pent-3-enyl. Beispiele für $C_2$-$C_6$-Hydroxyalkylgruppen sind$-CH_2CH_2OH$, $-CH_2CH_2CH_2OH$, $-CH_2CH(OH)CH_3$, $-CH_2CH_2CH_2CH_2OH$, wobei die Gruppe-$CH_2CH_2OH$ bevorzugt ist. Beispiele für $C_2$-$C_6$-Polyhydroxyalkylgruppen sind 1,2-Dihydroxyethyl, 2,3-Dihydroxypropyl, 3,4-Dihydroxybutyl und die 2,4-Dihydroxybutyl.

Bevorzugt ist es, wenn die Reste R1, R3 und R3 der Struktureinheit der allgemeinen Formel (I) unabhängig voneinander für eine Methylgruppe oder ein Wasserstoffatom stehen, insbesondere bevorzugt stehen R1, R2 und R3 für ein Wasserstoffatom.

Weiterhin ist es bevorzugt, wenn die Reste R4 und R6 der Struktureinheiten der allgemeinen Formeln (II) und (III) unabhängig voneinander für eine $C_1$-$C_6$-Alkylgruppe stehen. Insbesondere bevorzugt stehen sowohl R4 als auch R6 für eine Methylgruppe.

Die Reste R5, R5' und R5" der Struktureinheit der allgemeinen Formel (II) stehen bevorzugt unabhängig voneinander für eine $C_1$-$C_{20}$-Alkylgruppe. Besonders bevorzugt steht mindestens einer der Reste ausgewählt aus R5, R5' und R5" für eine Methylgruppe. Insbesondere bevorzugt ist es, wenn alle drei Reste R5, R5' und R5" für eine Methylgruppe stehen.

Der Rest R7 der Struktureinheit der allgemeinen Formel (III) steht bevorzugt für eine $C_2$-$C_6$-Hydroxyalkylgruppe. Besonders bevorzugt steht der Rest R7 für eine 2-Hydroxyethylgruppe, eine 2-Hydroxypropylgruppe, eine 3-Hydroxypropylgruppe, eine 2-Hydroxy-1-methylethylgruppe, eine 3-Hydroxy-2-methylpropylgruppe, eine 3-Hydroxy-1-methylpropylgruppe, eine 2-Hydroxy-1-methylpropylgruppe, eine 2-Hydroxybutylgruppe, eine 3-Hydroxybutylgruppe oder eine 4-Hydroxybutylgruppe. Insbesondere bevorzugt steht der Rest R7 der Struktureinheit der allgemeinen Formel (III) für eine 2-Hydroxypropylgruppe oder eine 2-Hydroxy-1-methylethylgruppe. Definitionsgemäß steht m für eine ganze Zahl von 2 bis 6. Es ist bevorzugt, wenn m für 2 oder 3 steht. Insbesondere bevorzugt steht m für 2.

Eine besonders bevorzugte Ausführungsform des ersten Erfindungsgegenstandes ist ein erfindungsgemäßes Mittel, welches dadurch gekennzeichnet ist, dass die Reste R1, R2 und R3 für ein Wasserstoffatom stehen, die Reste R4, R5, R5', R5" und R6 für eine Methylgruppe stehen, der Rest R7 für eine $C_2$-$C_6$-Hydroxyalkylgruppe steht und m gleich 2 oder 3 ist.

Bevorzugte und besonders bevorzugte Vertreter des im erfindungsgemäßen Mittel enthaltenen kationischen Acrylsäureester-Copolymers sind dadurch gekennzeichnet, dass sie mindestens eine Struktureinheit der allgemeinen Formel (I), mindestens eine Struktureinheit der allgemeinen Formel (II) und mindestens eine Struktureinheit der allgemeinen Formel (III) enthalten, wobei m gleich 2 oder 3 ist und die Reste R1, R2, R3, R4, R5, R5', R5", R6, R7 für eine der Substituentenkombinationen stehen, wie mit den Verbindungen Nr. 1 bis Nr. 333 in der Tabelle (beginnend auf S. 6) des Prioritätsdokumentes offenbart sind.

Als physiologisch verträgliche Gegenionen $X^-$ kommen beispielsweise Halogenidionen, Sulfationen, Hydrogensulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Die erfindungsgemäßen Mittel zum Färben und/oder Aufhellen von keratinischen Fasern enthalten das bzw. die kationische(n) Acrylsäureester-Copolymer(e) - bezogen auf ihr Gewicht - in einer Gesamtmenge von 0,001 bis 25 %, bevorzugt 0,01 bis 15 %, besonders bevorzugt 0,1 bis 10 % und insbesondere bevorzugt 0,5 bis 5 %.

Bei einem besonders bevorzugten kationischen Acrylsäureester-Copolymer handelt es sich um das unter dem INCI-Namen bekannte Polyquaternium-91.

Eine weitere besonders bevorzugte Ausführungsform des ersten Erfindungsgegenstandes ist daher ein Mittel zum Färben und/oder Aufhellen von keratinischen Fasern, welches dadurch gekennzeichnet ist, dass es als kationisches Acrylsäureester-Copolymer - bezogen auf sein Gewicht - 0,001 bis 25 %, bevorzugt 0,01 bis 15 %, besonders bevorzugt 0,1 bis 10 % und insbesondere bevorzugt 0,5 bis 5 % Polyquaternium-91 enthält.

Als zweiten wesentlichen Inhaltsstoff enthalten die erfindungsgemäßen Mittel mindestens eine farbverändernde Verbindung. Unter einer farbverändernden Verbindung wird im Rahmen der vorliegenden Erfindung eine Verbindung oder eine Substanz verstanden, mittels welcher die Farbe der keratinischen Fasern verändert werden kann. Von dieser Definition mit umfasst sind sowohl Verbindungen, welche die keratinische Fasern in Richtung eines dunkleren Farbtons verändern als auch Verbindungen, nach deren Anwendung die keratinischen Fasern einen helleren als den ursprünglichen Farbton aufweisen. Unter die Definition der farbverändernden Verbindung fallen Oxidationsfarbstoffvorprodukte, direktziehende Farbstoffe, Farbstoffvorstufen naturanaloger Farbstoffe und Oxidationsmittel.

Wenn das erfindungsgemäße Mittel neben dem kationischen Acrylsäureester-Copolymer als farbverändernde Verbindung mindestens ein Oxidationsfarbstoffvorprodukt und/oder mindestens einen direktziehenden Farbstoff enthält, so ist dies bevorzugt.

In einer weiteren bevorzugte Ausführungsform des ersten Erfindungsgegenstandes enthält das Mittel zum Färben und/oder Aufhellen von keratinischen Fasern daher als farbverändernde Verbindung mindestens ein Oxidationsfarbstoffvorprodukt und/oder mindestens einen direktziehenden Farbstoff.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Im Rahmen der zu dieser Erfindung führenden Arbeiten hat es sich gezeigt, dass die erfindungsgemäße Aufgabenstellung mit Mitteln enthaltend bestimmte Entwickler/Kuppler-Kombinationen in besonderem Maße erfüllt wird. Daher sind Mittel zum Färben von keratinischen Fasern, die neben dem erfindungsgemäßen kationischen Acrylsäureester-Copolymer bestimmte Kombinationen an Oxidationsfarbstoffvorprodukten enthalten, besonders bevorzugt.

Es ist bevorzugt, wenn die erfindungsgemäßen Mittel naben dem kationischen Acrylsäureester-Copolymer eine der nachfolgenden Entwickler/Kuppler-Kombinationen enthalten: p-Toluylendiamin / Resorcin; p-Toluylendiamin / 2-Methylresorcin; p-Toluylendiamin / 2-Amino-3-hydroxypyridin; p-Toluylendiamin / 2,7-Dihydroxynaphthalin; p-Toluylendiamin / 3-Aminophenol; p-Toluylendiamin / 1-Naphthol; p-Toluylendiamin / 1,5-Dihydroxynaphthalin; p-Toluylendiamin / 5-Amino-2-methylphenol; p-Toluylendiamin / 2-(2,4-Diaminophenoxy)ethanol; p-Toluylendiamin / 3-Amino-2-chlor-6-methylphenol; p-Toluylendiamin / 2,6-Dihydroxy-3,4-dimethylpyridin; p-Toluylendiamin / 3-Amino-2-methylamino-6-methoxypyridin; p-Toluylendiamin / 1,3-Bis(2,4-diaminophenyl)propan; 2,4,5,6-Tetraaminopyrimidin / Resorcin; 2,4,5,6-Tetraaminopyrimidin / 2-Methylresorcin; 2,4,5,6-Tetraaminopyrimidin / 2-Amino-3-hydroxypyridin; 2,4,5,6-Tetraaminopyrimidin / 2,7-Dihydroxy-naphthalin; 4,5-Diamino-1-(2-hydroxyethyl)pyrazol / Resorcin; 4,5-Diamino-1-(2-hydroxyethyl)-pyrazol / 2-Methylresorcin; 4,5-Diamino-1-(2-hydroxyethyl)pyrazol / 2-Amino-3-hydroxypyridin; 4,5-Diamino-1-(2-hydroxyethyl)pyrazol / 2,7-Dihydroxynaphthalin; p-Aminophenol / Resorcin; p-Aminophenol / 2-Methylresorcin; p-Aminophenol / 2-Amino-3-hydroxypyridin; p-Aminophenol / 2,7-Dihydroxynaphthalin; p-Aminophenol / 3-Aminophenol; p-Aminophenol / 1-Naphthol; p-Aminophenol / 1,5-Dihydroxynaphthalin; p-Aminophenol / 5-Amino-2-methylphenol; p-Aminophenol / 2-(2,4-Diaminophenoxy)ethanol; p-Aminophenol / 3-Amino-2-chlor-6-methylphenol; p-Aminophenol / 2,6-Dihydroxy-3,4-dimethylpyridin; p-Aminophenol / 3-Amino-2-methylamino-6-methoxypyridin; p-Aminophenol / 1,3-Bis(2,4-diaminophenyl)propan

Besonders bevorzugte erfindungsgemäße Mittel enthalten neben dem kationischen Acrylsäureester-Copolymer eine der nachfolgenden Kombinationen aus zwei Entwicklern und einem Kuppler: p-Toluylendiamin / 2,4,5,6-Tetraaminopyrimidin / Resorcin; p-Toluylendiamin / 2,4,5,6-Tetraaminopyrimidin / 2-Methylresorcin; p-Toluylendiamin / 2,4,5,6-Tetraaminopyrimidin / 2-Amino-3-hydroxypyridin; p-Toluylendiamin / 2,4,5,6-Tetraaminopyrimidin / 2,7-Dihydroxynaphthalin.

Es wird insbesondere dann eine besonders gute Farbintensivierung bzw. ein besonders guter Farberhalt erreicht, wenn die keratinischen Fasern mit einer Formulierung enthaltend die Kombination p-Toluylendiamin, 2,4,5,6-Tetraaminopyrimidin, Resorcin, 2-Methylresorcin, 2-Amino-3-hydroxypridin und 2,7-Dihydroxynaphthalin gefärbt werden.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Rahmen einer weiteren bevorzugten Ausführungsform enthält das erfindungsgemäße Mittel als farbverändernde Verbindung mindestens einen direktziehenden Farbstoff. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden, die entsprechend den Anforderungen der Trägerbasis vom Fachmann ausgewählt und eingesetzt werden.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Bevorzugt ist es, wenn das erfindungsgmäße Mittel als farbverändernde Verbindung den direktziehenden Farbstoff 4-Amino-3-nitrophenol entweder allein oder in Kombination mit weiteren farbverändernden Verbindungen enthält.

In einer weiteren bevorzugten Ausführungform enthalten die erfindungsgemäßen Mittel als farbverändernde Verbindungen sowohl einen direktziehenden Farbstoff als auch ein Oxidationsfarbstoffvorprodukt vom Entwicklertyp und/oder Kupplertyp.

Das erfindungsgemäße Mittel kann als farbverändernde Verbindung weiterhin mindestens eine Farbstoffvorstufe eines naturanalogen Farbstoffes enthalten. Als Farbstoffvorstufen naturanaloger Farbstoffe werden bevorzugt Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6dihydroxyindolin sowie 5,6-Dihydroxyindolin-2-carbonsäure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Das erfindungsgemäße Mittel enthält mindestens eine farbverändernde Verbindung jeweils in einem Gewichtsanteil von 0,001 bis 12 %. Handelt es sich bei dem farbverändernden Mittel um Oxidationsfarbstoffvorprodukte, direktziehende Farbstoffe und/oder Vorstufen naturanaloger Farbstoffe, so werden diese bevorzugt jeweils in einer Menge von 0,01 bis 10 Gew.-%, besonders bevorzugt von 0,1 bis 5 Gew.-% uns insbesondere bevorzugt von 0,25 bis 3 Gew.-% - bezogen auf das anwendungsbereite Mittel - eingesetzt.

Eine weitere besonders bevorzugte Ausführungsform des ersten Erfindungsgegenstandes ist daher ein Mittel zum Färben und/oder Aufhellen von keratinischen Fasern, welches dadurch gekennzeichnet ist, dass es als farbverändernde Verbindung mindestens ein Oxidationsfarbstoffvorprodukt und/oder mindestens einen direktziehenden Farbstoff, jeweils in einer Menge von 0,001 bis 12 Gew.-%, bevorzugt von 0,01 bis 10 Gew.-%, besonders bevorzugt von 0,1 bis 5 Gew.-% und insbesondere bevorzugt von 0,25 bis 3 Gew.-% - bezogen auf das anwendungsbereite Mittel - enthält.

Die erfindungsgemäßen Mittel können weiterhin als aufhellende Färbemittel oder als Aufhellmittel eingesetzt werden. Zur Erzielung des Aufhelleffekts enthalten die Mittel hierzu als farbverändernde Verbindung ein Oxidationsmittel. Bevorzugt wird als Oxidationsmittel Wasserstoffperoxid und/oder eines seiner festen Anlagerungsprodukte an organische oder anorganische Verbindungen verwendet. Beispiele für solche Anlagerungsprodukte sind an Anlagerungsprodukte an Harnstoff, Melamin sowie Natriumborat.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet.

Erfindungsgemäß bevorzugte anwendungsbereite Mittel des ersten Erfindungsgegenstands sind dadurch gekennzeichnet, dass sie das Oxidationsmittel in einer Menge von von 0,001 bis 12 Gew.-%, bevorzugt von 0,01 bis 10 Gew.-%, vorzugsweise 1 bis 9 Gew.-%, besonders bevorzugt 2,5 bis 8 Gew.-% und insbesondere 3 bis 6 Gew.-% - bezogen auf das anwendungsbereite Mittel - enthalten.

Eine weitere besonders bevorzugte Ausführungsform des ersten Erfindungsgegenstandes ist daher ein Mittel zum Färben und/oder Aufhellen von keratinischen Fasern, welches dadurch gekennzeichnet ist, dass es als farbverändernde Verbindung mindestens ein Oxidationsmittel, ausgewählt aus Wasserstoffperoxid und seinen festen Anlagerungsprodukten an organische oder anorganische Verbindungen, in einer Menge von 0,001 bis 12 Gew.-%, bevorzugt von 0,01 bis 10 Gew.-%, vorzugsweise 1 bis 9 Gew.-%, besonders bevorzugt 2,5 bis 8 Gew.-% und insbesondere 3 bis 6 Gew.-% - bezogen auf das anwendungsbereite Mittel - enthält.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

In einer weiteren bevorzugten Ausführunsform enthalten die erfindungsgemäßen Mittel daher als farbverändernde Verbindungen sowohl ein Oxidationsfarbstoffvorprodukt vom Entwicklertyp und/oder Kupplertyp als auch ein Oxidationsmittel.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Eine weitere bevorzugte Ausführungsform des ersten Erfindungsgegenstandes ist daher ein erfindungsgemäßes Mittel, welches dadurch gekennzeichnet ist, dass es zusätzlich - bezogen auf sein Gewicht - 0,01 bis 30 % eines Bleichkraftverstärkers, ausgewählt aus Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid, enthält.

Wenn die Mittel zusätzlich Persulfate enthalten, so sind diese Persulfate in dem Mittel zu 0,01 bis 30 % Gew.-%, bevorzugt zu 1,5 bis 28 Gew.-%, vorzugsweise 2,0 bis 25 Gew.-%, und insbesondere zu 5 bis 20 Gew.-% , jeweils bezogen auf das Gesamtgewicht des Mittels, enthalten.

Das Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine $SiO_2$-Verbindung, wie Kieselsäure oder Silicate, insbesondere Wassergläser, zugesetzt sein. Es kann erfin-

dungsgemäß bevorzugt sein, die $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-Verbindungen (ohne deren Wasseranteil) in den Mitteln wieder. Weiterhin hat es sich als vorteilhaft erweisen, wenn die Oxidationsmittelzubereitungen mindestens einen Stabilisator oder Komplexbildner enthalten. Gebräuchliche und im Rahmen der vorliegenden Erfindung bevorzugte Komplexbildner und Stabilisatoren sind beispielsweise Polyoxycarbonsäuren, Polyamine, Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfobernsteinsäure) (DDS), Ethylendicysteinsäure (EDC), Ethylendiamin-N-N'-bis(ortho-hydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate, geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate, Aminophosphonsäuren wie Ethylendiamintetra(methylenphosphonsäure) (EDTMP), Diethylentriaminpenta(methylenphosphonsäure) (DTPMP) sowie deren höhere Homologe, oder Nitrilotri(methylenphosphonsäure), Phosphonopolycarbonsäuren wie 2-Phosphonobutan-1,2,4-tricarbonsäure, Cyclodextrine, sowie Alkalistannate (Natriumstannat), Alkalipyrophosphate (Tetranatriumpyrophosphat, Dinatriumpyrophosphat), Alkaliphosphate (Natriumphosphat), und Phosphorsäure sowie deren Salze.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens ein erfindungsgemäßes kationisches Acrylsäureester-Copolymer enthält, und ein weiterer Container eine Oxidationsmittelzubereitung (B), enthaltend mindestens ein Oxidationsmittel, enthält. Gegebenenfalls zusätzlich enthaltende direktziehende Farbstoffe und/oder Oxidationsfarbstoffvorprodukte vom Entwickler und/oder Kupplertyp werden in diesem Fall vorteilthafter Weise zusammen mit dem erfindungsgemäßen kationisches Acrylsäureester-Copolymer in Mittel (A) konfektioniert.

In einer weiteren Ausführungsform der vorliegenden Erfindung sind Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens ein erfindungsgemäßes kationisches Acrylsäureester-Copolymer enthält, und ein weiterer Container ein Mittel (B), enthaltend mindestens eine farbverändernde Verbindung, enthält.

Die erfindungsgemäßen Mittel enthalten die kationischen Acrylsäureester-Copolymere und/oder die farbverändernde Verbindung(en) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tabletten-förmige Formulierung zu integrieren.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die Färbezubereitung und gegebenenfalls Oxidationsmittelzubereitung enthalten weitere Hilfs- und Zusatzstoffe. So hat es sich erfindungsgemäß als bevorzugt erwiesen, wenn die Färbezubereitung und/oder die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Im Rahmen der zu dieser Erfindung führenden Arbeiten hat sich gezeigt, dass vorteilhafte Ergebnisse insbesondere dann erhalten wurden, wenn das erfindungsgemäße Mittel zusätzlich mindestens ein anionisches Acrylsäurepolymer und/oder ein anionisches Acrylsäure-Copoylmer enthält.

Das anionische Acrylsäurepolymer und/oder anionische Acrylsäure-Copoylmer kann dabei in einem Gewichtsanteil von 0,001 bis 20 %, bevorzugt zu 0,01 bis 10 % und insbesondere bevorzugt von 0,5 bis 5 % im erfindungsgemäßen Mittel enthalten sein.

Eine weitere bevorzugte Ausführungsform des ersten Erfindungsgegenstandes ist daher ein erfindungsgemäßes Mittel, welches dadurch gekennzeichnet ist, dass es zusätzlich - bezogen auf sein Gewicht - 0,001 bis 20 % mindestens eines anionischen Acrylsäurepolymers und/oder ein anionisches Acrylsäure-Copolymers enthält.

In diesem Zusammenhang kann es bevorzugt sein, wenn es sich bei dem zusätzlich im erfindungsgemäßen Mittel enthaltenen anionische Acrylsäure-Copolymer um ein Copolymer handelt, welches durch Copolymerisation der Monomere Acrylsäure (Prop-2-ensäure), Methacrylsäure (2-Methylprop-2-ensäure), Acrylsäuremethylester (Methyl prop-2-enoat), Methacrylsäuremethylester (Methyl 2-methylprop-2-enoat), Acrylsäureethylester (Ethyl prop-2-enoat), Methacrylsäureethylester (Ethyl 2-methylprop-2-enoat), Acrylsäure-n-butylester (Butyl prop-2-enoat), Methacrylsäure-n-butylester (Butyl 2-methylprop-2-enoat), Ethen und/oder Styren (Ethenylbenzen) hergestellt wird. Gemäß einer bevorzugten Ausführungsform enthält das erfindungsgemäße Mittel zusätzlich ein anionisches Acrylsäure-Copolymer, welches durch Copolymerisation der Monomere Methacrylsäure (2-Methylprop-2-ensäure), Methacrylsäuremethylester (Methyl 2-methylprop-2-enoat), Acrylsäureethylester (Ethyl prop-2-enoat), Acrylsäure-n-butylester (Butyl prop-2-enoat), Ethen und/oder Styren (Ethenylbenzen) hergestellt wird.

Gemäß einer ganz besonders bevorzugten Ausführungsform enthält das erfindungsgemäße Mittel zusätzlich ein anionisches Acrylsäure-Copolymer, welches durch Copolymerisation der Monomere Methacrylsäure (2-Methylprop-2-ensäure), Methacrylsäuremethylester (Methyl 2-methylprop-2-enoat), Acrylsäureethylester (Ethyl prop-2-enoat), Acrylsäure-n-butylester (Butyl prop-2-enoat), Ethen und Styren (Ethenylbenzen) hergestellt wird.

Bei einem besonders bevorzugten anionischen Acrylsäure-Copolymer handelt es sich um das unter dem INCI-Namen bekannte Polyacrylat-15.

Eine weitere besonders bevorzugte Ausführungsform des ersten Erfindungsgegenstandes ist daher ein erfindungsgemäßes Mittel, welches dadurch gekennzeichnet ist, dass es als anionisches Acrylsäure-Copolymer Polyacrylat-15 enthält.

In einer weiteren Ausführungsform kann das erfindungsgemäße Mittel als anionische Polymere anionische Homopolymere enthalten. Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol® im Handel erhältlich. Ebenfalls bevorzugt ist das Homopolymer der 2-Acrylamido-2-methylpropansulfonsäure, das beispielsweise unter der Bezeichnung Rheothik®11-80 im Handel erhältlich ist.

Innerhalb einer weiteren Ausführungsform kann es ebenfalls bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester. Weitere bevorzugte anionische Copolymere sind beispielsweise Copolymere aus Acrylsäure, Methacrylsäure oder deren $C_1$-$C_6$-Alkylestern, wie sie unter der INCI-Deklaration Acrylates Copolymere vertrieben werden. Ein bevorzugtes Handelsprodukt ist beispielsweise Aculyn® 33 der Firma Rohm & Haas. Weiterhin bevorzugt sind aber auch Copolymere aus Acrylsäure, Methacrylsäure oder deren $C_1$-$C_6$-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn® 22 sowie von der Firma National Starch unter den Handelsbezeichnungen Structure® 2001 und Structure® 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz

kommen. Ein solches Polymer ist in den Handelsprodukten Sepigel®305 und Simulgel® 600 der Firma SEPPIC enthalten. Die Verwendung dieser Verbindungen, die neben der Polymerkomponente eine Kohlenwasserstoffmischung ($C_{13}$-$C_{14}$-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen. Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnung Stabileze® QM im Handel erhältlich.

Bevorzugt kann das erfindungsgemäße Mittel zusätzlich mindestens ein weiteres anionisches Acrylsäure- und/oder Methacrylsäure-Polymerisat oder -Copolymerisat enthalten. Bevorzugte Polymerisate dieser Art sind:

- Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren,
- Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll® D (BASF).
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Gemäß einer weiteren Ausführungsform handelt es sich bei dem Verdickungsmittel um ein kationisches synthetisches Polymer, das sich von den erfindungsgemäßen kationischen Polymeren unterscheidet. Bevorzugte kationische Polymere sind

- Homopolymere der allgemeinen Formel (HP-1),

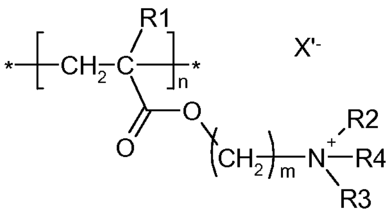

(HP-1)

in der R1 = -H oder -$CH_3$ ist, R2, R3 und R4 unabhängig voneinander ausgewählt sind aus $C_1$-$C_4$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sind besonders bevorzugte kationische polymere Gelbildner. Im Rahmen dieser Polymeren sind diejenigen erfindungsgemäß bevorzugt, für die mindestens eine der folgenden Bedingungen gilt:

- R1 steht für eine Methylgruppe
- R2, R3 und R4 stehen für Methylgruppen
- m hat den Wert 2.

Als physiologisch verträgliches Gegenionen $X'^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloxyethyltrimethylammoniumChlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6) im Handel erhältlich.

In einer weiteren bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar® C von der Firma Rhone Poulenc vertrieben. Erfindungsgemäß bevorzugte modifizierte Guargums enthalten $C_1$-$C_6$-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guargums sind unter den Handelsbezeichnungen Jaguar® HP8, Jaguar® HP60, Jaguar® HP120, Jaguar® DC 293 und Jaguar® HP105 der Firma Rhone Poulenc im Handel erhältlich.

Weiterhin geeignete natürliche Verdickungsmittel sind ebenfalls bereits aus dem Stand der Technik bekannt.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize® der Firma Amerchol und Natrosol® der Firma Hercules vertrieben werden. Geeignete Carboxyalkylcellulosen sind insbesondere die Carboxymethylcellulosen, wie sie unter den Bezeichnungen Blanose® von der Firma Aqualon, Aquasorb® und Ambergum® von der Firma Hercules und Cellgon® von der Firma Montello vertrieben werden.

Bevorzugt sind weiterhin Stärke und deren Derivate. Besonders vorteilhaft ist eine mit einer 2-Hydroxypropylgruppe veretherte Maisstärke, wie sie beispielsweise von der Firma National Starch unter der Handelsbezeichnung Amaze® vertrieben wird.

Aber auch nichtionische, vollsynthetische Polymere, wie beispielsweise Polyvinylalkohol oder Polyvinylpyrrolidinon, sind als erfindungsgemäße Verdickungsmittel einsetzbar. Bevorzugte nichtionische, vollsynthetische Polymere werden beispielsweise von der Firma BASF unter dem Handelsnamen Luviskol® vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate (polymere, kristalline Natriumdisilicate) als besonders geeignet im Sinne der vorliegenden Erfindung erwiesen.

Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel® vertriebene Magnesiumschichtsilikat, sind bevorzugt.

Hinsichtlich der gegebenenfalls hydratisierten $SiO_2$-Verbindungen sind Kieselsäuren, deren Oligomeren und Polymeren sowie deren Salze beovrzugt. Bevorzugte Salze sind die Alkalisalze, insbesondere die Kalium und Natriumsalze. Die Natriumsalze sind ganz besonders bevorzugt.

Die gegebenenfalls hydratisierten $SiO_2$-Verbindungen können in verschiedenen Formen vorliegen. Erfindungsgemäß bevorzugt werden die $SiO_2$-Verbindungen in Form von Kieselgelen (Silicagel) oder besonders bevorzugt als Wasserglas eingesetzt. Diese $SiO_2$-Verbindungen können teilweise in wässriger Lösung vorliegen. Erfindungsgemäß ganz besonders bevorzugt sind auch Wassergläser. Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben. Vorzugsweise wird dem erfindungsgemäßen Mittel weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl

Betaine bekannte Fettsäureamid-Derivat.

Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das $C_{12}$-$C_{18}$-Acylsarcosin.

Weiterhin hat es sich als vorteilhaft erwiesen, wenn die erfindungsgemäßen Färbe- und/oder Aufhellmittel weitere, nichtionogene grenzflächenaktive Stoffe, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 1 bis 50 Mol Ethylenoxid und/oder 0 bis 5 Mol Propylenoxid an lineare und verzweigte Fettalkohole mit 8 bis 30 C-Atomen, wie beispielsweise beispielsweise Lauryl-, Myristyl-, Cetyl-, aber auch Stearyl-, Isostearyl- und Oleylalkohol, an Fettsäuren mit 8 bis 30 C-Atomen und an Alkylphenole mit 8 bis 15 C-Atomen in der Alkylgruppe,
- mit einem Methyl- oder $C_2$-$C_6$-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- Polyglycerinester und alkoxylierte Polyglycerinester, wie beispielsweise Poly(3)glycerindiisostearat (Handelsprodukt: Lameform®TGI (Henkel)) und Poly(2)glycerinpolyhydroxy-stearat (Handelsprodukt: Dehymuls®PGPH (Henkel)).
- Polyolfettsäureester, wie beispielsweise das Handelsprodukt Hydagen® HSP (Cognis) oder Sovermol-Typen (Cognis),
- höher alkoxylierte, bevorzugt propoxylierte und insbesondere ethoxylierte, Mono-, Di- und Triglyceride, wie beispielsweise Glycerinmonolaurat + 20 Ethylenoxid und Glycerinmonostearat + 20 Ethylenoxid,
- Aminoxide,
- Hydroxymischether,
- Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO),
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylphenole und Alkylphenolalkoxylate mit 6 bis 21, insbesondere 6 bis 15 Kohlenstoffatomen in der Alkylkette und 1 bis 30 Ethylenoxid- und/oder Propylenoxid-Einheiten. Bevorzugte Vertreter dieser Klasse sind beispielsweise Nonylphenol + 9 EO und Octylphenol + 8 EO;
- Alkylpolyglykoside entsprechend der allgemeinen Formel RO-$(Z)_x$, wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Als nichtionische Tenside eignen sich insbesondere $C_8$-$C_{22}$-Alkylmono- und oligoglycoside und deren ethoxylierte Analoga. Insbesondere die nichtethoxylierten Verbindungen haben sich als besonders geeignet erwiesen.

Besonders bevorzugt sind solche Alkylpolyglykoside der Formel RO-$(Z)_x$, bei denen R

- im Wesentlichen aus $C_8$- und $C_{10}$-Alkylgruppen,
- im Wesentlichen aus $C_{12}$- und $C_{14}$-Alkylgruppen,
- im Wesentlichen aus $C_8$- bis $C_{16}$-Alkylgruppen oder
- im Wesentlichen aus $C_{12}$- bis $C_{16}$-Alkylgruppen oder
- im Wesentlichen aus $C_{16}$ bis $C_{18}$-Alkylgruppen besteht.

Diese Verbindungen sind dadurch gekennzeichnet, dass als Zuckerbaustein Z beliebige Mono- oder Oligosaccharide eingesetzt werden können. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine

sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus. Eine erfindungsgemäß besonders geeignete Verbindung aus dieser Substanzgruppe stellt das unter der Bezeichnung Tegoamid® S 18 im Handel erhältliche Stearamidopropyldimethylamin dar. Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart®C-4046, Dehyquart® L80 und Dehyquart® AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Ferner können die erfindungsgemäßen Mittel weitere Wirk-, Hilfs- und Zusatzstoffe enthalten, wie beispielsweise

- nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane;
- Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere mit nicht silikonhaltigem, organischen Grundgerüst oder mit Polysiloxan-Grundgerüst, wie beispielsweise das unter der INCI-Bezeichnung Bis-PEG/PPG-20/20 Dimethicone vertriebene Handelsprodukt Abil B 8832 der Firma Degussa, oder deren Gemischen;
- Strukturanten wie Glucose, Maleinsäure und Milchsäure,
- haarkonditionierende Verbindungen wie Phospholipide, beispielsweise Sojalecithin, Ei-Lecitin und Kephaline sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat

- Entschäumer wie Silikone,
- Farbstoffe zum Anfärben des Mittels,
- Antischuppenwirkstoffe wie Piroctone Olamine, Zink Omadine und Climbazol,
- Aminosäuren und Oligopeptide , insbesondere Arginin und/oder Serin,
- Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate,
- pflanzliche Öle, beispielsweise Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Panthenol, Pantothensäure, Pantolacton, Allantoin, Pyrrolidinoncarbonsäuren und deren Salze sowie Bisabolol,
- Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole;
- Ceramide, bevorzugt die Sphingolipide wie Ceramide I, Ceramide II, Ceramide 1, Ceramide 2, Ceramide 3, Ceramide 5 und Ceramide 6, oder Pseudoceramide, wie insbesondere N-($C_8$-$C_{22}$-Acyl)-($C_8$-$C_{22}$-acyl)-hydroxyprolin,
- Vitamine, Provitamine und Vitaminvorstufen, insbesondere solche der Gruppen A, $B_3$, $B_5$, $B_6$, C, E, F und H,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien.

Die Auswahl dieser weiteren Stoffe wird der Fachmann gemäß der gewünschten Eigenschaften der Mittel treffen.

Färbe- und Aufhellprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 11, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Ganz besonders bevorzugt sind Natriumhydroxid und/oder Kaliumhydroxid. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt. Schließlich ist ein weiteres bevorzugtes Alkalisierungsmittel Ammoniak.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Ein weiterer Gegenstand der Erfindung ist die Verwendung eines erfindungsgemäßen Mittels zum Färben und/oder Aufhellen von keratinhalten Fasern, insbesondere menschlichen Haaren. Insbesondere bevorzugt ist die Verwendung eines erfindungsgemäßen Mittels zur Verbesserung des Farbaufzugs von Färbemitteln auf keratinische Fasern und/oder zur Verbesserung der Waschstabilität von gefärbten keratinischen Fasern.

Zur Anwendung der erfindungsgemäßen Mittel eignet sich insbesondere ein Verfahren zum Färben und/oder Aufhellen von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die färbende und/oder aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Die erfindungsgemäßen Mittel können als Einkomponentenmittel oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt. Im Fall eines oxidativen Färbeverfahrens ist ein Färbe- und Aufhellverfahren, bei dem die Aufhellcreme und das Oxidationsmittel zunächst getrennt vorliegen, bevorzugt.

Ein weiterer Gegenstand der vorliegenden Erfindung ist daher ein Verfahren zum Färben und/oder Aufhellen von keratinischen Fasern, welches dadurch gekennzeichnet ist, dass

- gewünschtenfalls ein Vorbehandlungsmittel M1 auf die Faser aufgebracht wird, dann
- ein Färbe- und/oder Aufhellmittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von 2 - 25 Minuten wieder abgespült wird,

wobei das Mittel M2 ein erfindungsgemäßes Mittel ist.

Bezüglich weiterer bevorzugter Ausführungsformen der erfindungsgemäßen Verfahren und Verwendungen gilt mutatis mutandis das zu den erfindungsgemäßen Mitteln Gesagte.

Die folgenden Beispiele sollen den Gegenstand der vorliegenden Anmeldung näher erläutern ohne ihn jedoch in irgendeiner Form zu beschränken.

Ausführungsbeispiele

1. Intensivierung des Farbaufzugs

Es wurden die folgenden Rezepturen hergestellt. Die Mengenangaben verstehen sich, sofern nichts anderes vermerkt ist, jeweils in Gewichtsprozent. Bei V1 und V2 handelt es sich um Vergleichsrezepturen, E1 und E2 stellen die entsprechenden erfindungsgemäßen Formulierungen dar.

| Rohstoffe | V1 | E1 | V2 | E2 |
|---|---|---|---|---|
| Lanette D | 9,00 | 9,00 | 8,10 | 8,10 |
| Lorol tech. | 3,10 | 3,10 | 2,80 | 2,80 |
| Texapon NSO 27 %ig | 11,0 | 11,0 | 10,00 | 10,00 |
| Plantapon LGC | 5,55 | 5,55 | 5,00 | 5,00 |
| Eumulgin B1 | 0,28 | 0,28 | 0,25 | 0,25 |

(fortgesetzt)

| Rohstoffe | V1 | E1 | V2 | E2 |
|---|---|---|---|---|
| Ceteareth-20 | 0,50 | 0,50 | 0,25 | 0,25 |
| Iso-Stearinsäure | 2,22 | 2,22 | 2,00 | 2,00 |
| Myristinsäure 98-100 | 0,56 | 0,56 | 0,50 | 0,50 |
| Produkt W 37194 | 2,00 | 2,00 | 1,00 | 1,00 |
| Kaliumhydroxid 50 % | 1,63 | 1,63 | 1,20 | 1,20 |
| Syntran PC 5330 | --- | 5,00 | --- | 5,00 |
| p-Toluylendiamin, Sulfat | 1,15 | 1,15 | 1,08 | 1,08 |
| 2,4,5,6-Tetraaminopyrimidin, Sulfat | 1,10 | 1,10 | 1,02 | 1,02 |
| Resorcin | 0,14 | 0,14 | 0,11 | 0,11 |
| 2-Methylresorcin | 0,60 | 0,60 | 0,54 | 0,54 |
| 2-Amino-3-hydroxypyridin | 0,41 | 0,41 | 0,40 | 0,40 |
| 2,7-Dihydroxynaphthalin | 0,03 | 0,03 | 0,03 | 0,03 |
| 4-Amino-3-nitrophenol | 0,01 | 0,01 | --- | --- |
| 2-[(4-Amino-2-nitrophenyl)amino]benzoesäure | --- | --- | 0,09 | 0,09 |
| Natriumsulfit, wasserfrei, 96 % | 0,30 | 0,30 | 0,50 | 0,50 |
| Ascorbinsäure | 0,05 | 0,05 | 0,40 | 0,40 |
| Ammoniumsulfat techn. rein | 0,30 | 0,30 | 0,30 | 0,30 |
| Natriumsilikat 40/42 | 0,50 | 0,50 | 0,50 | 0,50 |
| 1-Hydroxyethan-1,1-diphosphonsäure 60 % | 0,20 | 0,20 | 0,20 | 0,20 |
| Ammoniak 25 % | 7,10 | 7,10 | 7,10 | 7,10 |
| Wasser | ad 100 | ad 100 | ad 100 | ad 100 |

Die Färbecremes V1 und E1 wurden unmittelbar vor der Anwendung mit der folgenden Oxidationsmittelzubereitung (Ox 1) im Verhältnis 1:1 vermischt:

| (Ox 1) Rohstoff | Menge |
|---|---|
| EDTA, Dinatriumsalz | 0,15 |
| Dinatriumpyrophosphat | 0,30 |
| Natriumbenzoat | 0,04 |
| Emulgade F | 2,10 |
| Wasserstoffperoxid 50 % | 12,00 |
| Wasser | ad 100 |

Die Färbecremes V2 und E2 wurden unmittelbar vor der Anwendung mit der folgenden Oxidationsmittelzubereitung (Ox 2) im Verhältnis 1:1 vermischt:

| (Ox 2) Rohstoff | Menge |
|---|---|
| Natronlauge 45 % techn. | 0,73 |
| Dipicolinsäure | 0,10 |
| Dinatriumpyrophosphat | 0,03 |

(fortgesetzt)

| (Ox 2) Rohstoff | Menge |
|---|---|
| 1-Hydroxyethan-1,1-diphosphonsäure 60 % | 1,50 |
| Texapon NSO 27 % | 2,00 |
| Dow Corning DB 110 A (nicht ionische Silikonemulsion) | 0,07 |
| Aculyn 33A | 12,00 |
| Wasserstoffperoxid 50 % | 12,00 |
| Wasser | ad 100 |

Verzeichnis der eingesetzten Rohstoffe

| | |
|---|---|
| Aculyn® 33A | ca. 28% Festkörper in Wasser; INCI-Bezeichnung: Acrylates Copolymer |
| Lanette® D | $C_{16-18}$-Fettalkohol (INCI-Bezeichnung: Cetearyl alcohol) (Cognis) |
| Lorol® tech. | $C_{12-18}$-Fettalkohol (INCI-Bezeichnung: Coconut alcohol) (Cognis) |
| Texapon® NSO | Laurylethersulfat, Natriumsalz (ca. 27,5% Aktivsubstanz; INCI-Bezeichnung: Sodium Laureth Sulfate) (Cognis) |
| Plantapon® LGC | ca. 28-34% Aktivsubstanzgehalt in Wasser; INCI-Bezeichnung: Lauryl Glucose Carboxylate, Lauryl Glucoside (Cognis) |
| Eumulgin®B 1 | Cetylstearylalkohol mit ca. 12-EO-Einheiten (INCI-Bezeichnung: Ceteareth-12) (Cognis) |
| Emulgade F | INCI-Bezeichnung: Cetearyl Alcohol, Alcohol, PEG-40 Castor Oil, Sodium Cetearylsulfate |
| Ceteareth-20 | Fettsäurealkohole, C16-C18, ethoxyliert (20 EO) |
| Produkt W 37194 | 1-Propanaminium, N,N,N-trimethyl-3-[(1-oxo-2-propenyl)amino]-, chloride, Polymer mit Natrium 2-Propenoat (INCI-Bezeichnung: Acrylamidopropyltrimoniumchloride/Acrylates Copolymer) |
| Syntran PC 5330 | Polyquaternium-91 (14 Gew.-%) und Polyacrylate-15 (4 Gew.-%) |

Vor dem Färbeprozeß wurden Haarsträhnen (Euro Naturhaar Weiß) farbmetrisch vermessen (Spectralflasch SF 450 Farbmetrik Gerät von Datacolor). Anschließend wurden die wie oben beschrieben hergestellten anwendungsbereiten Färbeformulierungen auf die Haarsträhnen gegeben und dort bei Raumtemperatur für 30 Minuten belassen. Anschließend wurden die Haarsträhnen gründlich ausgespült und im Luftstrom getrocknet. Nach dem Färben und Trocknen wurden die Haarsträhnen erneut farbmetrisch vermessen. Gemäß der nachfolgenden Formel wurde der Farbabstand (ΔE) zwischen ungefärbter und gefärbter Strähne berechnet:

$$\Delta E = \sqrt{(Lv - Ln)^2 + (av - an)^2 + (bv - bn)^2}$$

| | |
|---|---|
| *Lv*, a*v*, b*v* | Farbmetrikwerte vor dem Färben |
| Ln, a*n*, b*n* | Farbmetrikwerte nach dem Färben |

| Bestimmung des Farbaufzugsvermögens | | L | a | b | ΔE |
|---|---|---|---|---|---|
| Euronaturhaar weiß, ungefärbt | vor dem Färben | 70,50 | 2,55 | 21,11 | |
| Färbung mit V1 | nach dem Färben | 21,92 | 12,29 | 6,97 | 51,5 |
| Färbung mit E1 | nach dem Färben | 19,98 | 12,39 | 6,60 | 53,5 |

(fortgesetzt)

| Bestimmung des Farbaufzugsvermögens | | L | a | b | ΔE |
|---|---|---|---|---|---|
| Euronaturhaar weiß, ungefärbt | vor dem Färben | 70,50 | 2,55 | 21,11 | |
| Färbung mit V2 | nach dem Färben | 20,82 | 11,62 | 5,02 | 53,0 |
| Färbung mit E2 | nach dem Färben | 18,89 | 9,93 | 4,07 | 54,9 |

Je größer der Farbabstand zwischen ungefärbter Strähne und gefärbter Strähne ist, desto stärker ist der durch die Färbung resultierende Farbaufzug. Bei Anwendung der erfindungsgemäßen Formulierungen E1 und E2 wurde jeweils im Vergleich zur entsprechenden Vergleichsformulierung ein deutlich intensiveres Farbergebnis erhalten.

2. Verbesserung der Waschechtheit

Zur Bestimmung der Waschechtheiten bzw. des Farbrückhaltes wurden die mit den Rezepturen V2 und E2 unter Punkt 1. gefärbten Haarsträhnen in ein Ultraschallbad gegeben und nach einem standardisierten Verfahren 6, 12, 18 und 24 Haarwäschen unterzogen. Jeweils nach 6 Haarwäschen wurden die Strähnen aus dem Ultraschallbad entnommen, getrocknet und farbmetrisch vermessen.

Auf diese Weise wurden die farbmetrischen Daten direkt nach der Färbung der Haarsträhnen (0 HW) sowie nach 6, 12, 18 und 24 Haarwäschen bestimmt. Gemäß der nachfolgenden Formel wurde jeweils der Farbabstand (ΔE-Wert) zwischen der ungewaschenen und der definiert gewaschenen Strähne berechnet:

$$\Delta E = \sqrt{(L_0 - L_x)^2 + (a_0 - a_x)^2 + (b_0 - b_x)^2}$$

$L_0$, $a_0$, $b_0$ Farbmetrikwerte nach 0 Haarwäschen
$L_x$, $a_x$, $b_x$ Farbmetrikwerte jeweils nach 6, 12, 18 bzw. 24 Haarwäschen

| Bestimmung der Waschechtheiten | Haarwäschen | L | a | b | ΔE |
|---|---|---|---|---|---|
| V2 | 0 | 20,82 | 11,62 | 5,02 | |
| | 6 | 21,97 | 14,62 | 7,39 | 4,0 |
| | 12 | 21,75 | 14,35 | 7,08 | 3,5 |
| | 18 | 21,96 | 14,35 | 6,90 | 3,5 |
| | 24 | 23,40 | 15,70 | 8,96 | 6,2 |
| E2 | 0 | 18,89 | 9,93 | 4,07 | |
| | 6 | 19,43 | 11,95 | 5,50 | 2,5 |
| | 12 | 19,44 | 12,51 | 5,92 | 3,2 |
| | 18 | 19,44 | 12,65 | 6,08 | 3,4 |
| | 24 | 19,88 | 13,11 | 6,56 | 4,2 |

Je größer der Farbabstand (ΔE-Wert) zwischen ungewaschener und gewaschener Strähne ist, desto schlechter ist deren Waschechtheit. Ein Vergleich der mit den Rezepturen V2 und E2 gefärbten Strähnen zeigt, dass die mit der erfindungsgemäßen Rezeptur E2 gefärbten Strähnen bei allen Waschzyklen einen kleineren Farbabstand und damit eine bessere Waschechtheit aufweisen.

**Patentansprüche**

1. Mittel zum Färben und/oder Aufhellen von keratinischen Fasern, insbesondere menschlichen Haaren, enthaltend in einem kosmetischen Träger

(a) mindestens ein kationisches Acrylsäureester-Copolymer enthaltend mindestens eine Struktureinheit der allgemeinen Formel (I), mindestens eine Struktureinheit der allgemeinen Formel (II) und mindestens eine Struktureinheit der allgemeinen Formel (III),

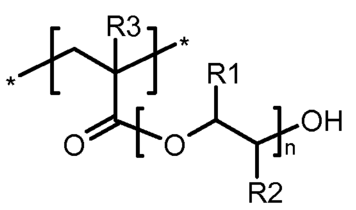

(I)

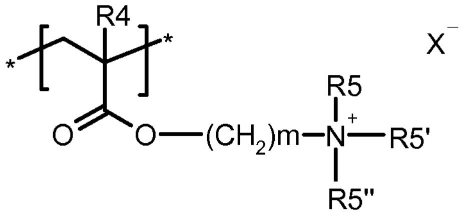

(II)

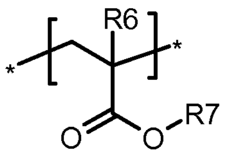

(III)

worin

R1, R2, R3, R4, R6 unabhängig voneinander für ein Wasserstoffatom oder eine $C_1$-$C_6$-Alkylgruppe stehen,
R5, R5', R5" unabhängig voneinander für eine $C_1$-$C_{20}$-Alkylgruppe, eine $C_2$-$C_6$-Alkenylgruppe oder eine $C_2$-$C_6$-Hydroxyalkylgruppe stehen,
R7 für eine $C_1$-$C_6$-Alkylgruppe, eine $C_2$-$C_6$-Hydroxyalkylgruppe oder eine $C_2$-$C_6$-Polyhydroxyalkylgruppe steht,
n für eine ganze Zahl von 1 bis 50 000 steht,
m für eine ganze Zahl von 2 bis 6 steht,
$X^-$ für ein physiologisch verträgliches Anion steht, und

(b) mindestens eine farbverändernde Verbindung.

2. Mittel nach Anspruch 1, **dadurch gekennzeichnet, dass** die Reste R1, R2 und R3 für ein Wasserstoffatom stehen, die Reste R4, R5, R5', R5" und R6 für eine Methylgruppe stehen, der Rest R7 für eine $C_2$-$C_6$-Hydroxyalkylgruppe steht und m gleich 2 oder 3 ist.

3. Mittel nach einem der Ansprüche 1 oder 2, **dadurch gekennzeichnet, dass** es als kationisches Acrylsäureester-Copolymer - bezogen auf sein Gewicht - 0,001 bis 25 %, bevorzugt 0,01 bis 15 %, besonders bevorzugt 0,1 bis 10 % und insbesondere bevorzugt 0,5 bis 5 % Polyquaternium-91 enthält.

4. Mittel nach einem der Ansprüche 1 bis 3, **dadurch gekennzeichnet, dass** es als farbverändernde Verbindung mindestens ein Oxidationsfarbstoffvorprodukt und/oder mindestens einen direktziehenden Farbstoff, jeweils in einer Menge von 0,001 bis 12 Gew.-%, bevorzugt von 0,01 bis 10 Gew.-%, besonders bevorzugt von 0,1 bis 5 Gew.-% und insbesondere bevorzugt von 0,25 bis 3 Gew.-% - bezogen auf das anwendungsbereite Mittel - enthält.

5. Mittel nach einem der Ansprüche 1 bis 4, **dadurch gekennzeichnet, dass** es als farbverändernde Verbindungen eine der nachfolgenden Entwickler / Kuppler-Kombinationen enthält: p-Toluylendiamin / Resorcin; p-Toluylendiamin / 2-Methylresorcin; p-Toluylendiamin / 2-Amino-3-hydroxypyridin; p-Toluylendiamin / 2,7-Dihydroxynaphthalin; p-Toluylendiamin / 3-Aminophenol; p-Toluylendiamin / 1-Naphthol; p-Toluylendiamin / 1,5-Dihydroxynaphthalin; p-Toluylendiamin / 5-Amino-2-methylphenol; p-Toluylendiamin / 2-(2,4-Diaminophenoxy)ethanol; p-Toluylendiamin / 3-Amino-2-chlor-6-methylphenol; p-Toluylendiamin / 2,6-Dihydroxy-3,4-dimethylpyridin; p-Toluylendiamin / 3-Amino-2-methylamino-6-methoxypyridin; p-Toluylendiamin / 1,3-Bis(2,4-diaminophenyl)propan; 2,4,5,6-Tetraaminopyrimidin / Resorcin; 2,4,5,6-Tetraaminopyrimidin / 2-Methylresorcin; 2,4,5,6-Tetraaminopyrimidin / 2-Amino-3-hydroxypyridin; 2,4,5,6-Tetraaminopyrimidin / 2,7-Dihydroxynaphthalin; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 2-Amino-3-hydroxypyridin; 4,5-Diamino-1-(2-hydroxyethyl)pyrazol / 2,7-Dihydroxy-naphthalin; p-Aminophenol / Resorcin; p-Aminophenol / 2-Methylresorcin; p-Aminophenol / 2-Amino-3-hydroxypyridin; p-Aminophenol / 2,7-Dihydroxynaphthalin; p-Aminophenol / 3-Aminophenol; p-Aminophenol / 1-Naphthol; p-Aminophenol / 1,5-Dihydroxynaphthalin; p-Aminophenol / 5-Amino-2-methylphenol; p-Aminophenol / 2-(2,4-Diaminophenoxy)ethanol; p-Aminophenol / 3-Amino-2-chlor-6-methylphenol; p-Aminophenol / 2,6-Dihydroxy-3,4-dimethylpyridin; p-Aminophenol / 3-Amino-2-methylamino-6-methoxypyridin; p-Aminophenol / 1,3-Bis(2,4-diaminophenyl)propan.

6. Mittel nach einem der Ansprüche 1 bis 5, **dadurch gekennzeichnet, dass** es als farbverändernde Verbindungen eine der nachfolgenden Kombinationen aus zwei Entwicklern und einem Kuppler enthält: p-Toluylendiamin / 2,4,5,6-Tetraaminopyrimidin / Resorcin; p-Toluylendiamin / 2,4,5,6-Tetraaminopyrimidin / 2-Methylresorcin; p-Toluylendiamin / 2,4,5,6-Tetraaminopyrimidin / 2-Amino-3-hydroxypyridin; p-Toluylendiamin / 2,4,5,6-Tetraaminopyrimidin / 2,7-Dihydroxynaphthalin.

7. Mittel nach einem der Ansprüche 1 bis 6, **dadurch gekennzeichnet, dass** es als farbverändernde Verbindung einen nichtionischen direktziehenden Farbstoff aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methyl-benzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

8. Mittel nach einem der Ansprüche 1 bis 7, **dadurch gekennzeichnet, dass** es als farbverändernde Verbindung den direktziehenden Farbstoff 4-Amino-3-nitrophenol entweder allein oder in Kombination mit weiteren farbverändernden Verbindungen enthält.

9. Mittel nach einem der Ansprüche 1 bis 8, **dadurch gekennzeichnet, dass** es als farbverändernde Verbindungen sowohl einen direktziehenden Farbstoff als auch ein Oxidationsfarbstoffvorprodukt vom Entwicklertyp und/oder Kupplertyp enthält.

10. Mittel nach einem der Ansprüche 1 bis 9, **dadurch gekennzeichnet, dass** es als farbverändernde Verbindung mindestens ein Oxidationsmittel, ausgewählt aus Wasserstoffperoxid und seinen festen Anlagerungsprodukten an organische oder anorganische Verbindungen, in einer Menge von 0,001 bis 12 Gew.-%, bevorzugt von 0,01 bis 10 Gew.-%, vorzugsweise 1 bis 9 Gew.-%, besonders bevorzugt 2,5 bis 8 Gew.-% und insbesondere 3 bis 6 Gew.-% - bezogen auf das anwendungsbereite Mittel - enthält.

11. Mittel nach einem der Ansprüche 1 bis 10, **dadurch gekennzeichnet, dass** es zusätzlich - bezogen auf sein Gewicht - 0,01 bis 30 % eines Bleichkraftverstärkers, ausgewählt aus Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid, enthält.

12. Mittel nach einem der Ansprüche 1 bis 11, **dadurch gekennzeichnet, dass** es zusätzlich - bezogen auf sein Gewicht - 0,001 bis 20 % mindestens eines anionischen Acrylsäurepolymers und/oder ein anionisches Acrylsäure-Copolymers enthält.

13. Mittel nach Anspruch 12, **dadurch gekennzeichnet, dass** es als anionisches Acrylsäure-Copolymer Polyacrylat-15 enthält.

14. Verwendung eines Mittels nach einem der Ansprüche 1 bis 13 zur Verbesserung des Farbaufzugs von Färbemitteln auf keratinische Fasern und/oder zur Verbesserung der Waschstabilität von gefärbten keratinischen Fasern.

15. Verfahren zum Färben und/oder Aufhellen von keratinischen Fasern, **dadurch gekennzeichnet, dass**

- gewünschtenfalls ein Vorbehandlungsmittel M1 auf die Faser aufgebracht wird, dann
- ein Färbe- und/oder Aufhellmittel M2 auf der Faser zur Anwendung kommt, wobei gewünschtenfalls dem Mittel M2 vor der Anwendung ein weiteres Mittel M3 zugegeben wird,

- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von 2 - 25 Minuten wieder abgespült wird,

wobei das Mittel M2 ein erfindungsgemäßes Mittel nach einem der Ansprüche 1 bis 13 ist.

## Claims

**1.** An agent for dyeing and/or brightening keratin fibers, in particular human hair, containing

(a) at least one cationic acrylic acid ester copolymer containing at least one structural unit of the general formula (I), at least one structural unit of the general formula (II) and at least one structural unit of the general formula (III),

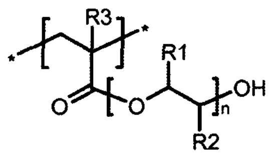

(I)

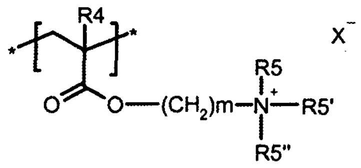

(II)

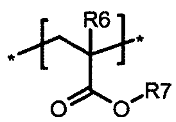

(III)

where

$R^1$, $R^2$, $R^3$, $R^4$ and $R^6$ represent, independently of one another, a hydrogen atom or a $C_1$-$C_6$ alkyl group,
$R^5$, $R^{5'}$ and $R^{5''}$ represent, independently of one another, a $C_1$-$C_{20}$ alkyl group, a $C_2$-$C_6$ alkenyl group or a $C_2$-$C_6$ hydroxyalkyl group,
$R^7$ represents a $C_1$-$C_6$ alkyl group, a $C_2$-$C_6$ hydroxyalkyl group or a $C_2$-$C_6$polyhydroxyalkyl group,
n represents an integer from 1 to 50,000,
m represents an integer from 2 to 6,
$X^-$ represents a physiologically acceptable anion, and

(b) at least one color-changing compound in a cosmetic carrier.

**2.** The agent according to claim 1, **characterized in that** the functional groups $R^1$, $R^2$ and $R^3$ represent a hydrogen atom, the functional groups $R^4$, $R^5$, $R^{5'}$, $R^{5''}$ and $R^6$ represent a methyl group, the functional group $R^7$ represents a $C^2$-$C^6$ hydroxyalkyl group and m is equal to 2 or 3.

**3.** The agent according to one of claims 1 or 2, **characterized in that** said agent contains, based on its weight, from 0.001 to 25 %, preferably from 0.01 to 15 %, more preferably from 0.1 to 10 % and most preferably from 0.5 to 5 % polyquaternium-91 as the cationic acrylic acid ester copolymer.

**4.** The agent according to one of claims 1 to 3, **characterized in that** said agent contains, as the color-changing compound, at least one oxidation dye precursor and/or at least one direct dye, each in an amount of from 0.001 to 12 wt.%, preferably from 0.01 to 10 wt.%, more preferably from 0.1 to 5 wt.% and most preferably from 0.25 to 3 wt.%, based on the ready-to-use agent.

**5.** The agent according to one of claims 1 to 4, **characterized in that** said agent contains one of the following developer/coupler combinations as the color-changing compounds: p-toluylenediamine / resorcinol; p-toluylenediamine / 2-methylresorcinol; p-toluylenediamine / 2-amino-3-hydroxypyridine; p-toluylenediamine / 2,7-dihydroxynaphthalene; p-toluylenediamine / 3-aminophenol; p-toluylenediamine / 1-naphthol; p-toluylenediamine / 1,5-dihydroxynaphthalene; p-toluylenediamine / 5-amino-2-methylphenol; p-toluylenediamine / 2-(2,4-diaminophenoxy)ethanol; p-toluylenediamine / 3-amino-2-chloro-6-methylphenol; p-toluylenediamine / 2 ,6-dihydroxy-3,4-dimethylpyridine; p-toluylenediamine / 3-amino-2-methyl-amino-6-methoxypyridine; p-toluylenediamine / 1,3-bis(2,4-diaminophenyl)propane; 2,4,5,6-tetraaminopyrimidine / resorcinol; 2,4,5,6-Tetraaminopyrimidine / 2-methylresorcinol; 2,4,5,6-tetraaminopyrimidine / 2-amino-3-hydroxypyridine; 2,4,5,6-tetraaminopyrimidine / 2,7-di-hydroxynaphthalene; 4,5-diamino-1-(2-hydroxyethyl)pyrazole / resorcinol; 4,5-diamino-1-(2-hydroxyethyl)pyrazole / 2-methylresorcinol; 4,5-diamino-1-(2-hydroxyethyl)pyrazole / 2-amino-3-hydroxypyridine; 4,5-diamino-1-(2-hydroxyethyl)pyrazole/2,7-dihydroxynaphthalene; p-aminophenol / resorcinol; p-aminophenol / 2-methylresorcinol; p-aminophenol / 2-amino-3-hydroxypyridine; p-aminophenol / 2,7-dihydroxynaphthalene; p-aminophenol / 3-aminophenol; p-aminophenol / 1-naphthol; p-aminophenol / 1,5-dihydroxynaphthalene; p-aminophenol / 5-amino-2-methylphenol; p-aminophenol / 2-(2,4-diamino-phenoxy)ethanol; p-aminophenol / 3-amino-2-chloro-6-methylphenol; p-aminophenol / 2,6-dihydroxy-3,4-dimethylpyridine; p-aminophenol / 3-amino-2-methylamino-6-methoxypyridine; p-aminophenol / 1,3-bis(2,4-diaminophenyl)propane.

6. The agent according to one of claims 1 to 5, **characterized in that** said agent contains one of the following combinations of two developers and one coupler as the color-changing compounds: p-toluylenediamine / 2,4,5,6-tetraaminopyrimidine / resorcinol; p-toluylenediamine / 2,4,5,6-tetraaminopyrimidine / 2-methylresorcinol; p-toluylenediamine / 2,4,5,6-tetraaminopyrimidine / 2-amino-3-hydroxypyridine; p-toluylenediamine / 2,4,5,6-tetraaminopyrimidine / 2,7-dihydroxynaphthalene.

7. The agent according to one of claims 1 to 6, **characterized in that** said agent contains, as the color-changing compound, a non-ionic direct dye from the group comprising HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-diamino-2-nitrobenzene, 2-amino-4-nitrophenol, 1,4-bis-(2-hydroxyethyl)amino-2-nitrobenzene, 3-nitro-4-(2-hydroxyethyl)aminophenol, 2-(2-hydroxyethyl)amino-4,6-dinitrophenol, 4-[(2-hydroxyethyl)amino]-3-nitro-1-methylbenzene, 1-amino-4-(2-hydroxyethyl)amino-5-chloro-2-nitrobenzene, 4-amino-3-nitrophenol, 1-(2'-ureidoethyl)amino-4-nitrobenzene, 2-[(4-amino-2-nitrophenyl)amino]benzoic acid, 6-nitro-1,2,3,4-tetrahydroquinoxaline, 2-hydroxy-1,4-naphthoquinone, picramic acid and the salts thereof, 2-amino-6-chloro-4-nitrophenol, 4-ethylamino-3-nitrobenzoic acid and 2-chloro-6-ethylamino-4-nitrophenol.

8. The agent according to one of claims 1 to 7, **characterized in that** said agent contains the direct dye 4-amino-3-nitrophenol as the color-changing compound, either on its own or in combination with additional color-changing compounds.

9. The agent according to one of claims 1 to 8, **characterized in that** said agent contains, as the color-changing compounds, both a direct dye and an oxidation dye precursor in the form of a developer and/or coupler.

10. The agent according to one of claims 1 to 9, **characterized in that** said agent contains, as the color-changing compound, at least one oxidizing agent selected from hydrogen peroxide and the solid addition products thereof of organic or inorganic compounds, in an amount of from 0.001 to 12 wt.%, preferably from 0.01 to 10 wt.%, preferably from 1 to 9 wt.%, more preferably from 2.5 to 8 wt.%, and in particular from 3 to 6 wt.%, based on the ready-to-use agent.

11. The agent according to one of claims 1 to 10, **characterized in that** said agent additionally contains, based on its weight, from 0.01 to 30 % of a bleaching power enhancer selected from ammonium peroxodisulfate, potassium peroxodisulfate, sodium peroxodisulfate, potassium hydrogen peroxomonosulfate, potassium peroxodiphosphate, magnesium peroxide and barium peroxide.

12. The agent according to one of claims 1 to 11, **characterized in that** said agent additionally contains, based on its weight, from 0.001 to 20 % of at least one anionic acrylic acid polymer and/or one anionic acrylic acid copolymer.

13. The agent according to claim 12, **characterized in that** said agent contains polyacrylate-15 as the anionic acrylic acid copolymer.

14. The use of an agent according to one of claims 1 to 13 for improving the color absorption of dyes on keratin fibers and/or for improving the wash stability of dyed keratin fibers.

**15.** A method for dyeing and/or brightening keratin fibers, **characterized in that**

- a pretreatment agent M1 is applied to the fiber if desired, then
- a dye and/or brightening agent M2 is used on the fiber, an additional agent M3 being added, if desired, to the agent M2 before said agent is used,
- said agent M2 is rinsed off the fiber after 5-30 minutes,
- and a post-treatment agent M4 is optionally applied to the fiber after treatment and is rinsed off again after an exposure time of 2-25 minutes, the agent M2 being an agent according to the invention according to one of claims 1 to 13.

## Revendications

**1.** Produit de coloration et/ou d'éclaircissement de fibres de kératine, en particulier de cheveux humains, contenant dans un vecteur cosmétique :

a) au moins un copolymère cationique d'un ester d'acide acrylate contenant au moins une unité structurelle de formule générale (I), au moins une unité structurelle de formule générale (II) et au moins une unité structurelle de formule générale (III),

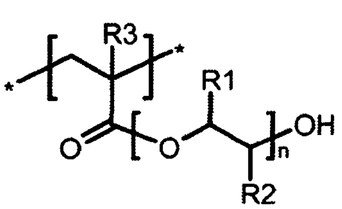

(I)

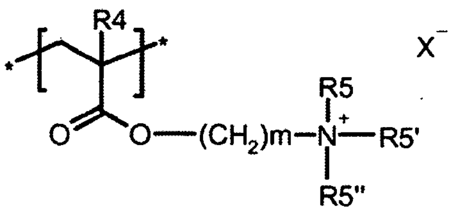

(II)

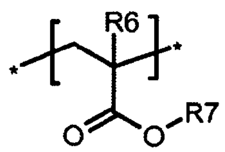

(III)

où

$R^1$, $R^2$, $R^3$, $R^4$, $R^6$ représentent indépendamment les uns des autres un atome d'hydrogène ou un groupe $C_{1-6}$-alkyle,
$R^5$, $R^{5'}$, $R^{5''}$ représentent indépendamment les uns des autres un groupe $C_{1-20}$-alkyle, un groupe $C_{2-6}$-alcényle ou un groupe $C_{2-6}$-ou hydroxyalkyle,
$R^7$ représente un groupe $C_{1-6}$-alkyle, un groupe $C_{2-6}$-hydroxyalkyle un groupe poly-$C_{2-6}$-hydroxyalkyle,
n est un entier entre 1 et 50.000,
m est un entier entre 2 et 6,
$X^-$ est un anion physiologiquement compatible, et

b) au moins un composant modificateur de coloration.

**2.** Produit selon la revendication 1, **caractérisé en ce que** les résidus $R^1$, $R^2$ et $R^3$ représentent un atome d'hydrogène, les $R^4$, $R^5$, $R^{5'}$, $R^{5''}$ et $R^6$ représentent un groupe méthyle, le résidu $R^7$ représente un groupe $C_{2-6}$-hydroxyalkyle, et m = 2 ou 3.

**3.** Produit selon la revendication 1 ou 2, **caractérisé en ce qu'**il contient comme copolymère cationique d'un ester d'acide acrylate - rapporté à son poids - 0,001 à 25 %, préférentiellement 0,01 à 15 %, particulièrement préférentiellement 0,1 à 10 % et en particulier 0,5 à 5 % de polyquaternium 91.

4. Produit selon une des revendications 1 à 3, **caractérisé en ce qu'**il contient comme composant modificateur de coloration au moins un précurseur de coloration oxydante et/ou au moins un colorant direct, respectivement à hauteur de 0,001 à 12 % en poids, préférentiellement de 0,01 à 10 % en poids, particulièrement préférentiellement de 0,1 à 5 % en poids et en particulier de 0,25 à 3 % en poids rapporté au produit prêt à l'emploi.

5. Produit selon une des revendications 1 à 4, **caractérisé en ce qu'**il contient comme composants modificateurs de coloration une des combinaisons développeur / coupleur suivantes : paratoluylène diamine / résorcinol, paratoluylène diamine / 2-méthylrésorcinol, paratoluylène diamine / 2-amino-3-hydroxypyridine, paratoluylène diamine / 2,7-dihydroxyphtaline, paratoluylène diamine / 3-aminophénol, paratoluylène diamine / 1-naphtol, paratoluylène diamine / 1,5-dihydroxynaphtaline, paratoluylène diamine / 5-amino-2-méthylphénol, paratoluylène diamine / 2-(2,4-diaminophénoxy)éthanol, paratoluylène diamine / 3-amino-2-chloro-6-méthylphénol, paratoluylène diamine / 2,6-dihydroxy-3,4-diméthylpyridine, paratoluylène diamine / 3-amino-2-méthylamino-6-méthoxypyridine, paratoluylène diamine / 1,3-bis-(2,4-diaminophényl)propane, 2,4,5,6-tétraaminopyrimidine / résorcinol, 2,4,5,6-tétraaminopyrimidine / 2-méthylrésorcinol, 2,4,5,6-tétraaminopyrimidine / 2-amino-3-hydroxypyridine, 2,4,5,6-tétraaminopyrimidine / 2,7-dihydroxyphtaline, 4,5-diamino-1-(2-hydroxyéthyl)pyrazol / résorcinol, 4,5-diamino-1-(2-hydroxyéthyl)pyrazol / 2-méthylrésorcinol, 4,5-diamino-1-(2-hydroxyéthyl)pyrazol / 2-amino-3-hydroxypyridine, 4,5-diamino-1-(2-hydroxyéthyl)pyrazol / 2,7-dihydroxynaphtaline, paraaminophénol / résorcinol, paraaminophénol / 2-méthylrésorcinol, paraaminophénol / 2-amino-3-hydroxypyridine, paraaminophénol / 2,7-dihydroxyphtaline, paraaminophénol / 3-aminophénol, paraaminophénol / 1-naphtol, paraaminophénol / 1,5-dihydroxynaphtaline, paraaminophénol / 5-amino-2-méthylphénol, paraaminophénol / 2-(2,4-diaminophénoxy)éthanol, paraaminophénol / 3-amino-2-chloro-6-méthylphénol, paraaminophénol / 2,6-dihydroxy-3,4-diméthylpyridine, paraaminophénol / 3-amino-2-méthylamino-6-méthoxypyridine, paraaminophénol / 1,3-bis-(2,4-diaminophényl)propane.

6. Produit selon une des revendications 1 à 5, **caractérisé en ce qu'**il contient comme composants modificateurs de coloration une des combinaisons suivantes de deux développeurs et d'un coupleur :

   paratoluylène diamine / 2,4,5,6-tétraaminopyrimidine / résorcinol, paratoluylène diamine / 2,4,5,6-tétraaminopyrimidine / 2-méthylrésorcinol, paratoluylène diamine / 2,4,5,6-tétraaminopyrimidine / 2-amino-3-hydroxypyridine, paratoluylène diamine / 2,4,5,6-tétraaminopyrimidine / 2,7-dihydroxynaphtaline.

7. Produit selon une des revendications 1 à 6, **caractérisé en ce qu'**il contient comme composant modificateur de coloration un colorant direct non-ionique issu du groupe constitué de HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 11, HC Blue 12, Disperse Blue 3, HC Violet 1, Disperse Violet 1, Disperse Violet 4, Disperse Black 9, 1,4-diamino-2-nitrobenzène, 2-amino-4-nitrophénol, 1,4-bis-(2-hydroxyéthyl)amino-2-nitrobenzène, 3-nitro-4-(2-hydroxyéthyl)aminophénol, 2-(2-hydroxyéthyl)amino-4,6-dinitrophénol, 4-((2-hydroxyéthyl)amino]-3-nitro-1-méthylbenzène, 1-amino-4-(2-hydroxyéthyl)amino-5-chloro-2-nitrobenzène, 4-amino-3-nitrophénol, 1-(2'-uréidoéthyl)amino-4-nitrobenzène, acide 2-[(4-amino-2-nitrophényl)amino]benzylique, 6-nitro-1,2,3,4-tétrahydroquinoxaline, 2-hydroxy-1,4-naphtoquinone, acide picraminique et ses sels, 2-amino-6-chloro4-nitrophénol, acide 4-éthylamino-3-nitrobenzoïque et 2-chloro-6-éthylamino-4-nitrophénol.

8. Produit selon une des revendications 1 à 7, **caractérisé en ce qu'**il contient comme composant modificateur de coloration le colorant direct 4-amino-3-nitrophénol, soit seul, soit en association avec d'autres composés modificateurs de coloration.

9. Produit selon une des revendications 1 à 8, **caractérisé en ce qu'**il contient comme composants modificateurs de coloration un colorant direct aussi bien un colorant direct qu'un précurseur de coloration oxydante de type développeur et/ou de type coupleur.

10. Produit selon une des revendications 1 à 9, **caractérisé en ce qu'**il contient comme composant modificateur de coloration au moins un oxydant choisi parmi le peroxyde d'hydrogène et ses produits d'addition solides avec des composés organiques ou minéraux, à hauteur de 0,001 à 12 % en poids, préférentiellement de 0,01 à 10 % en poids, préférentiellement de 1 à 9 % en poids, particulièrement préférentiellement de 2,5 à 8 % en poids et en particulier de 3 à 6% en poids, rapporté au produit prêt à l'emploi.

11. Produit selon une des revendications 1 à 10, **caractérisé en ce qu'**il contient en plus, rapporté à son poids, 0,01 à 30 % d'un intensificateur de blanc choisi parmi le peroxodisulfate d'ammonium, le peroxodisulfate de potassium, le peroxodisulfate de sodium, l'hydrogénopersulfate de potassium, le peroxodiphosphate de potassium, le peroxyde

de magnésium ou le peroxyde de baryum.

**12.** Produit selon une des revendications 1 à 11, **caractérisé en ce qu'**il contient en plus, rapporté à son poids, 0,001 à 20 % d'au moins un polymère anionique de l'acide acrylique et/ou un copolymère anionique de l'acide acrylique.

**13.** Produit selon la revendication 12 **caractérisé en ce qu'**il contient comme copolymère anionique de l'acide acrylique du polyacrylate 15.

**14.** Utilisation d'un produit selon une des revendications 1 à 13 pour améliorer l'enrobage de fibres de kératine par des colorants et/ou pour améliorer la stabilité au lavage de fibres de kératine colorées.

**15.** Procédé de coloration et/ou éclaircissement de fibres de kératine, **caractérisé en ce que** :

- si on le désire, on applique un produit de préparation M1 sur la fibre, puis
- on utilise un colorant et/ou un éclaircissant M2 sur la fibre, à quoi on ajoute au produit M2, si on le désire, un autre produit M3 avant utilisation,
- on rince ce produit M2 au bout de 5 à 30 minutes, et
- après le traitement, on applique éventuellement un produit après-traitement M4 sur la fibre, et on rince à nouveau après une durée d'action de 2 à 25 minutes, le produit M2 étant un produit selon l'invention selon une des revendications 1 à 13.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 7066966 B
- US 7147672 B
- US 20110219552 A1
- US 20110044924 A


### In der Beschreibung aufgeführte Nicht-Patentliteratur


- Silicones Used in Permanent and Semi-Permanent Hair Dyes to Reduce the Fading and Color Change Process of Dyed Hair Occuring by Wash-Out or UV Radiation. *J. Cosmetic Sci.,* 2004, vol. 55, 123-131
- **KH. SCHRADER.** Grundlagen und Rezepturen der Kosmetika. Hüthig Buch Verlag, 1989